# FASCICULE DE BREVET EUROPEEN

(11) **EP 1 895 851 B1**
(45) Date de publication et mention de la délivrance du brevet: **19.10.2016**
(21) Numéro de dépôt: 06763756.1
(22) Date de dépôt: 15.06.2006
(51) Int. Cl.: A23C 9/137, A23C 19/076, A23C 9/13, A23C 19/09

(54) **PRODUITS LAITIERS FRAIS A POUVOIR SATIETOGENE ET PROCEDES DE PREPARATION**
FRISCHE MILCHPRODUKTE MIT SÄTTIGUNGSVERMÖGEN SOWIE VERFAHREN ZU IHRER HERSTELLUNG
FRESH DAIRY PRODUCTS WITH SATIETOGENIC POWER AND METHODS FOR PREPARING SAME

(30) Priorité: 17.06.2005 FR 0506192
(43) Date de publication de la demande: 12.03.2008
(73) Titulaire: COMPAGNIE GERVAIS DANONE, 75009 Paris (FR)
(72) Inventeur: AYMARD, Pierre, F-92160 Antony (FR); LLUCH, Anne, F-91120 Palaiseau (FR); ARNOULT DELEST, Véronique, F-92160 Antony (FR); SCHMITT, Laurent, F-91430 Igny (FR); SANCHEZ HEREDERO, Antonio, E-08025 Barcelone (ES); SOLER BADOSA, Jose-Enrique, E-08025 Barcelone (ES); CARLSEN, Deanna, Texas, TX 76107 (US); LEBAS, Gilles, F-75016 Paris (FR); LYOTHIER, Arnaud, F-92600 Asnières (FR); BERTHET, Benoit, F-91120 Palaiseau (FR); DELOFFRE, Fabienne, F-75014 Paris (FR)
(74) Mandataire: Regimbeau
(86) Numéro de dépôt international: PCT/EP2006/063263
(87) Numéro de publication internationale: WO 2006/134159

(56) Documents cités:
- EP-A- 0 257 941
- EP-A- 0 290 054
- EP-A- 0 887 020
- DE-A1- 19 943 188
- US-A- 4 178 390
- US-A- 4 851 239
- US-A1- 2002 119 948
- US-A1- 2005 175 761
- HALL W L ET AL: "Casein and whey exert different effects on plasma amino acid proffies, gastrointestinal hormone secretion and appetite", BRITISH JOURNAL OF NUTRITION, CAMBRIDGE UNIVERSITY PRESS, CAMBRIDGE, GB, vol. 89, no. 2, 1 February 2003 (2003-02-01), pages 239-248, XP002546619, ISSN: 0007-1145, DOI: 10.1079/BJN2002760
- BOIRIE YVES ET AL: "SLOW AND FAST DIETARY PROTEINS DIFFERENTLY MODULATE POSTPRANDIAL PROTEIN ACCRETION", PROCEEDINGS OF THE NATIONAL ACADEMY OF SCIENCES, NATIONAL ACADEMY OF SCIENCES, US, vol. 94, 1 December 1997 (1997-12-01), pages 14930-14935, XP002099786, ISSN: 0027-8424, DOI: 10.1073/PNAS.94.26.14930
- "SIMPLESSE R - FIRST PRODUCTS LAUNCHED IN THE UK", CONFECTIONERY PRODUCTION, SPECIALISED PUBLICATIONS LTD., SURREY, GB, vol. 58, no. 3, 1 March 1992 (1992-03-01), pages 208-209,211, XP000264178, ISSN: 0010-5473

## Description

La présente invention se rapporte à un procédé de fabrication d'un produit laitier frais pauvre en matières grasses et en sucres, à faible densité énergétique, comprenant une ou plusieurs protéines satiétogènes. La ou lesdites protéines comprennent des protéines laitières sériques, le cas échéant, associées à des fibres alimentaires hydrosolubles et de préférence des fibres alimentaires hydrosolubles viscosifiantes.

L'augmentation de l'incidence de l'obésité et de ses complications est aujourd'hui telle que l'Organisation Mondiale de la Santé, dans son rapport «Diet, nutrition and the ,prevention of chronic diseases» de mars 2004, estime qu'il s'agit d'une évolution épidémique. Son coût social et économique rend urgente la mise en oeuvre d'actions devant s'appuyer tant sur l'éducation des consommateurs et la mise en place de règles de communication que sur l'amélioration nutritionnelle des produits manufacturés.
Bien que les causes identifiées soient multiples (alimentation trop riche en énergie, sédentarité ou manque d'activité physique, influence de la télévision et de la publicité...), l'industrie alimentaire est fréquemment incriminée. On reproche notamment aux industriels de fournir aux consommateurs des produits trop gras, trop sucrés, trop salés, et surtout trop appétents, d'où une difficulté des consommateurs pour réguler efficacement leur prise alimentaire.
La gestion du poids passe d'abord par l'équilibre entre les apports et les dépenses énergétiques. Un moyen efficace pour réguler les apports consiste donc à bien contrôler la prise alimentaire. A ce titre, la littérature scientifique fait aujourd'hui état de preuves qui attestent que certains aliments, de part leur teneur en nutriments, peuvent jouer un rôle plus ou moins favorable sur la satiété et, ainsi, sur le contrôle de la prise alimentaire.
Dans ce contexte, il existe une réelle demande des consommateurs pour des produits destinés à les aider à gérer leur poids, avec notamment un meilleur contrôle des sensations de faim (entraînant par exemple un retard dans l'apparition de la sensation de faim entre deux prises alimentaires). Typiquement, de tels produits s'adressent aux consommateurs soucieux de leur ligne, qui généralement font des efforts, ou souhaitent en faire, pour réduire leur prise alimentaire, mais qui souffrent notamment de l'apparition plus ou moins récurrente et tenace de sensations de faim en cas de régime alimentaire, ces sensations étant bien souvent responsables de l'échec des régimes.

La satiété est définie par l'absence de signal de faim, lequel, lorsqu'il est présent, incite à rechercher et à consommer de la nourriture. Après un repas ou une prise alimentaire, l'aliment ingéré provoque une réduction progressive de l'état de faim pour conduire finalement à un arrêt total de la prise alimentaire. Cet effet est médié par un processus complexe, impliquant d'abord des effets sensoriels puis cognitifs, pré-absorptifs et enfin post-absorptifs des aliments. L'ensemble de ce processus est décrit dans la cascade de la satiété proposée par JE Bundell (Green et al, 1997).
L'état de satiété résulte en fait de conditions métaboliques dans lesquelles les cellules de l'organisme (et tout particulièrement certaines cellules de l'hypothalamus) continuent de disposer de la capacité à oxyder le glucose disponible en quantité adéquate pour satisfaire leurs besoins métaboliques. Ce principe est le fondement de la "théorie glucostatique du contrôle de la prise alimentaire", formulée dès 1953 par Jean Mayer qui proposa l'hypothèse selon laquelle « *the short-term articulation between energy needs and energy intake was under glucosfatic control »* (ce qui se traduit par : « l'articulation à court terme entre les besoins et les apports énergétiques est sous un contrôle glucostatique »). Depuis, de nombreux arguments scientifiques complémentaires ont renforcé la validité de cette hypothèse (Louis-Sylvestre & Le Magnen, 1980 ; Melanson et al, 1999), et ce, même si d'autres théories sont également proposées.
Selon l'hypothèse susmentionnée, la faim résulte donc de la baisse de la disponibilité intracellulaire du glucose. Cependant, étant donné les délais d'absorption intestinale des nutriments, en particulier du glucose, l'arrêt de l'ingestion ne peut être relié directement aux nutriments consommés au cours du repas. Il doit donc aussi faire appel à des mécanismes physiologiques distincts qualifiés de « mécanismes du rassasiement ». Le rassasiement détermine la quantité d'aliments (ou d'énergie) consommée au cours du repas, quantité qui est inconsciemment évaluée par le cerveau du sujet grâce à l'ensemble des stimulations orales, gastriques et intestinales associées à l'ingestion et en résultant (Booth, 1985).

Si l'on s'intéresse aux facteurs capables de moduler la satiété, il faut se rappeler que la durée de l'état de satiété dépend de l'utilisation du glucose disponible, qui elle-même dépend de l'utilisation des autres nutriments. C'est ainsi que la composition en macronutriments des aliments ingérés et/ou leurs propriétés physico-chimiques sont susceptibles d'influencer la vitesse d'absorption digestive, et par là-même l'utilisation métabolique de ces aliments. Ces caractéristiques du produit ingéré constituent donc autant de facteurs susceptibles de modifier la durée de la satiété induite par celui-ci.

Parmi les méthodes de mesure du rassasiement et de la satiété, l'on distingue généralement les méthodes de mesure de marqueurs comportementaux d'une part, et les méthodes de mesure de marqueurs périphériques d'autre part. En outre, des méthodes de mesure de marqueurs centraux ont également été rapportées. Le Tableau 1 ci-dessous résume les marqueurs les plus courants. Pour plus d'informations sur ces marqueurs, voir la revue de De Graaf et al, 2004.

**Tableau 1**

| MARQUEURS | RASSASIEMENT (fin du repas) | SATIETE (commencement du repas) |
|---|---|---|
| Comportementaux | Prise alimentaire | Prise alimentaire précédente |
| | | Intervalle de temps entre les repas |
| | Détermination de l'appétit subjectif (e.g., faim et sensation de plénitude gastrique) | Détermination dé l'appétit subjectif (e.g., faim et sensation de plénitude gastrique) |
| Périphériques | Distension stomachale | Evolution du taux plasmatique de glucose dans le sang (ST) |
| | Mesure de la CCK plasmatique (Cholescystokinine) | Mesure de la Leptine (LT) plasmatique |
| | Mesure du GLP-1 (Glucagon-like peptide-1) plasmatique | Mesure de la ghréline (ST & LT) plasmatique |
| Centraux | Image du cerveau | Image du cerveau |

| | | |
|---|---|---|
| ST: à court terme LT: à long terme | | |

La littérature rapporte un certain nombre de recherches conduites chez l'homme avec des aliments de teneurs variables en protéines, glucides et lipides. Ces études ont permis de mesurer l'effet satiétogène relatif de ces divers macronutriments (Poppitt et al, 1998; Westererp-Plantega et al, 1999 ; Araya et al, 2000 ; Warwick et al, 2000). D'après les conclusions de ces travaux,
- les protéines possèdent l'effet le plus important sur la satiété et le rassasiement ;
- la teneur en lipides de l'aliment ne semble pas avoir d'effet significatif sur la satiété ;
- la teneur en glucides exerce un effet modéré tant sur la satiété que sur le rassasiement.

Il est important de souligner que l'ensemble des travaux effectues à ce jour ne permet cependant pas de tirer de conclusions très précises, en particulier quant aux effets spécifiques de telles ou telles protéines, des différents types d'acides gras ou encore des glucides (par exemple en fonction de leur index glycémique). En particulier, des travaux conduits en 1999 chez l'animal ont montré la supériorité des protéines sur les glucides, mais la nature des protéines (protéines de lait comparées au gluten) était restée sans effet (Bensaid et al, 2002). La littérature ne permet pas non plus de définir les teneurs précises en ces nutriments à atteindre dans les aliments pour obtenir l'effet souhaité. Une revue publiée en avril 2004 (Anderson & Moore, 2004) confirme le rôle des protéines dans la régulation des apports alimentaires chez l'homme, notamment grâce à leurs effets sur la satiété.

En ce qui concerne l'effet potentiel des fibres alimentaires, différents travaux (Delargy et al, 1997 ; Burton-Freeman, 2000 ; Holt et al, 2001 ; Howarth et al, 2001) s'accordent à dire que la teneur et la nature des fibres d'un aliment concourent à augmenter son caractère rassasiant et satiétogène, principalement par deux mécanismes :
- l'augmentation du temps de mastication et de la distension gastrique, surtout pour les fibres insolubles , et
- le ralentissement du temps de vidange gastrique et de l'absorption intestinale des nutriments, surtout pour les fibres hydrosolubles viscosifiantes.

En particulier, parmi les nombreuses fibres alimentaires étudiées par les Inventeurs dans le cadre de la présente invention, la gomme de guar a constitué une piste d'investigation particulièrement sérieuse. La plupart des travaux rapportés dans l'état de la technique montrent en effet qu'une dose de 2 g environ de gomme de guar par prise d'un produit alimentaire permet de moduler les sensations subjectives de satiété chez un individu consommant ce produit.

Il est important de signaler qu'à ce jour, la notion de « fibres alimentaires » est définie de manière différente en fonction des pays. Ainsi, au Royaume-Uni, le Comité sur les Aspects Santé des Aliments (Committee on Medical aspects of Foods, COMA) a donné en 1998 une définition très restrictive : « Dietary fiber is non-starch polysaccharide as measured by the Englyst method », ce qui se traduit par « les fibres alimentaires sont des polysaccharides non amylacés mesurés par la méthode Englyst ». La méthode Englyst, reconnue comme méthode de référence au Royaume-Uni par la UK Food Standard Agency, dose, en tant que fibres alimentaires, tous les polysaccharides non amylacés, à l'exclusion des fructo-oligosaccharides et des composés non glucidiques (lignines, tannins,..). Dans le reste de l'Europe, la méthode de référence est l'AOAC 985.29 (AOAC : Association of Analytical Communities) qui reconnaît, en tant que fibres alimentaires, les polysaccharides et composés non glucidiques ainsi que la fraction insoluble des fructo-oligosaccharides. Une définition plus large encore des fibres alimentaires est fournie par la Société Américaine des Chimistes des Céréales (American Association of Cereal Chemists, AACC, 2000) : « les fibres alimentaires sont une partie comestible d'origine végétale ou des glucides analogues qui ne sont pas digérés ni absorbés dans l'intestin grêle et partiellement ou complètement fermentés dans le côlon. Les fibres incluent des polysaccharides, oligosaccharides, la lignine et autres substances végétales».

Il est donc précisé qu'aux fins de la présente invention, les fibres alimentaires répondent à la définition large de tous les composés pouvant être dosés comme fibres par une méthode appropriée (fibres totales par la méthode AOAC 985.29, fructo-oligosaccharides par la méthode AOAC 997.08). De telles fibres sont des :
- polysaccharides de réserve comme les glucomannanes extraites des graines de konjac, galactomannanes issus des graines de guar, caroube, karaya, tracaganth et fenugrec ;
- polysaccharides de structure (présents dans les parois végétales) comme les pectines, alginates, carraghénanes ;
- polysaccharides produits par fermentation bactérienne comme le xanthane, gellane, ... ;
- exsudats végétaux (gomme acacia, extraits de mélèze) ;
- oligofructoses ou fructooligosaccharides extraits à partir de chicorée ;
- polymères de synthèse comme le polydextrose.
Les fibres mises en oeuvre dans la présente invention sont reconnues comme fibres par les trois définitions.

Dès lors, à la lumière des informations disponibles à ce jour dans la littérature, les protéines et/ou les fibres alimentaires semblent donc présenter un intérêt notable aux fins de la formulation d'un produit alimentaire à pouvoir satiétogène.

On notera du reste qu'au-delà de la composition nutritionnelle des aliments, leurs caractéristiques physico-chimiques ont également un impact sur leurs propriétés satiétogènes et rassasiantes.
Ainsi, la densité énergétique des aliments semble être un facteur défavorable selon une étude récente montrant que plus la teneur en lipides (donc en calories) augmentait, plus l'apport énergétique des sujets était important (Green et al, 2000). Cependant, d'après la plupart des études, il demeure difficile de conclure sur l'effet spécifique de la densité calorique car, lorsqu'elle varie, le volume consommé varie également (Bell et al, 1998 ; Araya et al, 1999). Ceci est confirmé par différents travaux de B. Rolls (Rolls et al, 1999 ; 2000) montrant que la modification du volume d'une précharge grâce à de l'air ou de l'eau réduisait significativement la faim et l'énergie consommée au repas suivant.
L'effet de la texture des aliments solides a été globalement peu étudié. Dans la revue de Howarth et al (2001), il est rapporté que l'augmentation du temps de mastication et, de ce fait, l'augmentation des secrétions salivaires (par exemple avec les fibres) pouvait avoir un impact favorable sur le rassasiement. De plus, des différences de texture peuvent aussi avoir des conséquences métaboliques, par exemple sur le niveau de sécrétion d'insuline (Labouré et al, 2002).
En outre, au-delà de la texture, l'effet de la viscosité des aliments est aussi un facteur important. Les travaux de Marciani et al (2001) et Mattes et al (2001) ont montré qu'une viscosité élevée favorisait d'abord un bon rassasiement (probablement par un effet volume) et, ensuite, une bonne satiété (probablement par un effet sur la vidange gastrique).

Dès lors, aux fins du développement d'un procédé pour la fabrication d'un produit laitier satiétogène, les Inventeurs ont cherché à multiplier les effets en tentant d'associer un enrichissement en protéines et/ou en fibres alimentaires à des modifications de texture et une augmentation de viscosité. En effet, ces différents éléments, ainsi combinés les uns aux autres, seraient avantageusement de nature à favoriser l'apparition de la satiété. Toutefois, de telles associations imposent des contraintes technologiques difficiles à concilier en pratique, ce qui explique l'absence globale, sur le marché actuel, de produits satiétogènes, notamment dans la gamme des produits laitiers frais, combinant de manière satisfaisante tout ou partie de ces effets.

Aussi existe-t-il un besoin pour des produits alimentaires, notamment des produits laitiers frais, pauvres en matières grasses et en sucres, peu caloriques, dotés d'un pouvoir satiétogène, et dont les caractéristiques organoleptiques sont satisfaisantes pour le consommateur tout en restant compatibles avec des procédés de fabrication de produits laitiers frais standard.

Dans le contexte de l'invention, les « produits laitiers frais » désignent plus particulièrement des produits laitiers frais et fermentés, prêts à la consommation humaine, c'est-à-dire des aliments laitiers frais et fermentés. Dans la présente demande, sont plus particulièrement visés les laits fermentés et yoghourts. Lesdits aliments laitiers frais et fermentés peuvent alternativement être des fromages blancs ou des petits-suisses. On donne aux termes « laits fermentés » et « yoghourts » leurs significations usuelles dans le domaine de l'industrie laitière, c'est-à-dire des produits qui sont destinés à la consommation humaine et qui sont issus de la fermentation lactique acidifiante d'un substrat laitier. Ces produits peuvent contenir des ingrédients secondaires tels que fruits, végétaux, sucre, etc. On peut, par exemple, se reporter au Décret français n° 88-1203 du 30 décembre 1988 relatif aux laits fermentés et au yaourt ou yoghourt, publié au Journal Officiel de la République Française du 31 décembre 1988.
On peut également se reporter au « Codex Alimentarius » (préparé par la Commission du Codex Alimentarius sous l'égide de la FAO et de l'OMS, et publié par la Division Information de la FAO, disponible en ligne sur http://www.codexalimentarius.net; cf. plus particulièrement le volume 12 du Codex Alimentarius « Normes Codex pour le lait et les produits laitiers », et la norme « CODEX STAN A -1 1(a)-1975 »).
L'expression « lait fermenté » est ainsi réservée dans la présente demande au produit laitier préparé avec un substrat laitier qui a subi un traitement au moins équivalent à la pasteurisation, ensemencé avec des microorganismes appartenant à l'espèce ou aux espèces caractéristique(s) de chaque produit. Un « lait fermenté » n'a subi aucun traitement permettant de soustraire un élément constitutif du substrat laitier mis en oeuvre et n'a notamment pas subi un égouttage du coagulum. La coagulation des « laits fermentés » ne doit pas être obtenue par d'autres moyens que ceux qui résultent de l'activité des microorganismes utilisés.

Le terme « yoghourt » est quant à lui réservé au lait fermenté obtenu, selon les usages locaux et constants, par le développement des bactéries lactiques thermophiles spécifiques dites *Lactobacillus bulgaricus* et *Streptococcus thermophilus,* qui doivent se retrouver vivantes dans le produit fini, à raison d'au moins 10 millions de bactéries par gramme rapportées à la partie lactée.

Dans certains pays, la réglementation autorise l'ajout d'autres bactéries lactiques dans la production de yoghourt, et notamment l'utilisation additionnelle de souches de *Bifidobacterium* et/ou *Lactobacillus acidophilus* et/ou *Lactobacillus casei.* Ces souches lactiques additionnelles sont destinées à conférer au produit fini diverses propriétés, telles que celle de favoriser l'équilibre de la flore intestinale ou de moduler le système immunitaire.

Dans la pratique, l'expression « lait fermenté » est donc généralement utilisée pour désigner les laits fermentés autres que les yoghourts. Elle peut d'ailleurs prendre, selon les pays, des noms aussi divers que, par exemple, « Kefir », « Kumiss », « Lassi », « Dahi », « Leben », « Filmjôlk », « Villi », « Acidophilus milk ».

S'agissant des laits fermentés, la quantité d'acide lactique libre contenue dans le substrat laitier fermenté ne doit pas être inférieure à 0,6 g pour 100 g lors de la vente au consommateur, et la teneur en matière protéique apportée à la partie lactée ne doit pas être inférieure à celle d'un lait normal.

Enfin, la dénomination « fromage blanc » ou « petit-suisse » est, dans la présente demande, réservée à un fromage non affiné, non salé, qui a subi une fermentation par des bactéries lactiques uniquement (et aucune autre fermentation que la fermentation lactique).

La teneur en matière sèche des fromages blancs peut être abaissée jusqu'à 15 g ou 10 g pour 100 g de fromage blanc, selon que leur teneur en matières grasses est supérieure à 20 g, ou au plus égale à 20 g pour 100 g de fromage blanc, après complète dessication. La teneur en matière sèche d'un fromage blanc est comprise entre 13 et 20 %. La teneur en matière sèche d'un petit-suisse quant à elle n'est pas inférieure à 23 g pour 100 g de petit-suisse. Elle est généralement comprise entre 25 et 30%.

L'on entend ici par « procédé de fabrication standard », un procédé mettant en oeuvre des étapes et équipements pour l'essentiel simples et/ou conventionnels. De préférence, un procédé dit « standard » répond aux exigences rencontrées de manière générale dans l'industrie alimentaire et, plus particulièrement, dans l'industrie laitière, à savoir : (i) une gestion globale des coûts satisfaisante ; (ii) des temps de fabrication des produits assez courts (notion de rendement) ; (iii) une « différentiation retardée » consistant à utiliser aussi longtemps que possible la même chaîne de fabrication pour des produits finals présentant des caractéristiques différentes (teneurs en ingrédients, types d'ingrédients, ...), notamment en partant d'une même masse blanche dans laquelle sont incorporées une ou plusieurs préparations intermédiaires contenant des ingrédients plus spécifiques au produit final plus tardivement au cours du procédé ; (iv) en relation avec la « différentiation retardée », l'absence de contamination des chaînes de fabrication par des ingrédients particuliers aussi longtemps que possible durant le procédé de fabrication, une contamination obligeant sinon à procéder à des étapes de nettoyage fastidieuses et parfois longues qui généralement retardent l'obtention du produit fini ; (v) l'absence de contamination microbiologique des produits aux différents stades du procédé ; (vi) un temps de stockage des produits, intermédiaires et finals, assez long ; et (vii) une « convivialité » pour le fabricant qui manipule les produits, c'est-à-dire, s'agissant par exemple de produits laitiers frais, ils doivent être injectables et/ou pompables, tout en étant également « conviviaux » pour le consommateur (les produits doivent présenter l'onctuosité et la cuillérabilité attendues s'il s'agit de yoghourts ou fromages frais ; ou être suffisamment onctueux tout en restant liquides s'il s'agit de yoghourts à boire).

Toutes ces contraintes, plus ou moins inconciliables en pratique, ont été étudiées par les Inventeurs qui sont, non sans beaucoup de difficultés et de manière inespérée, parvenus à mettre au point des moyens, notamment des procédés, qui permettent de répondre de manière tout à fait satisfaisante au besoin actuel.

La présente invention décrit donc un procédé selon la revendication 1.

Le terme « satiétogène » tel qu'utilisé ici répond aux définitions communément admises dans le domaine. Cette notion fait d'ailleurs l'objet d'un nombre croissant de publications. A toutes fins utiles, on précise que par « produit ou protéine satiétogène », l'on entend ici un produit ou une protéine qui, chez le consommateur, entraîne notamment une diminution des sensations de faim, une diminution de l'appétit, une augmentation de la plénitude gastrique, un retard dans le retour de la faim entre deux prises alimentaires, une prolongation de l'intervalle entre deux prises alimentaires, une diminution des apports alimentaires après ingestion. Ces différents effets peuvent être observés isolément ou associés, en tout ou en partie. On rappelle en outre qu'il existe des méthodes de mesure de marqueurs qui permettent de déterminer le pouvoir satiétogène d'une protéine ou d'un produit, comme cela est décrit plus haut (voir notamment le Tableau 1 supra). En particulier, des protéines satiétogènes contribuent à la libération de signaux prié- et post-absorptifs qui participent au contrôle de la cinétique gastrique, de la sécrétion pancréatique et des apports alimentaires. Les actions de ces signaux jouent au niveau périphérique et central (voir Tableau 1 supra).

Plus particulièrement, le ou lesdites protéines satiétogènes sont mises en oeuvre dans le produit de manière à ralentir sa métabolisation. On définit ici un « ralentissement de la métabolisation » d'un produit comme étant un ralentissement et/ou un retard dans la digestion et/ou dans l'absorption et/ou dans l'assimilation dudit produit.

Au sens de l'invention, un produit est « pauvre en matières grasses » s'il contient :
- moins de 3 g environ de matières grasses pour 100 g de produit lorsque le produit est solide (du type yoghourt ferme ou fromage frais) ;
- moins de 1,5 g environ de matières grasses pour 100 ml de produit lorsqu'il est liquide (du type yoghourt à boire).
A cet égard, la Demanderesse précise que la définition ci-dessus est conforme aux directives du Codex pour l'utilisation des allégations nutritionnelles (Codex Guidelines for the Use of Nutrition Claims) adoptées par la Commission Codex Alimentarius en 1997 et modifiées en 2001.

Un produit « pauvre en sucres » ou « à faible teneur en sucres » est tel qu'il ne contient pas plus de :
- 0,5 g de sucres environ pour 100 g de produit s'il est solide ;
- 2,5 g de sucres environ pour 100 ml s'il est liquide.
Là encore, la Demanderesse fait observer que cette définition est conforme à l'Avis de la Commission interministérielle d'étude des produits destinés à une alimentation particulière, en date du 8 juillet 1998 et relatif au caractère non trompeur des seuils des allégations nutritionnelles.

Par produit « à faible densité énergétique », l'on entend ici un produit apportant de 40 à 120 kcal environ pour 100 g, de préférence de 60 à 110 kcal environ pour 100 g, de préférence encore de 70 à 100 kcal environ pour 100 g.

L'expression « facteur d'enrichissement du produit en protéines satiétogènes allant de 2 à 5 environ par rapport à la teneur en protéines dans le produit initial » signifie que l'on ajoute des protéines satiétogènes à hauteur d'environ 2 à 5 fois la quantité de protéines contenue dans le produit initial.

Au sens de l'invention, un « produit initial » est un produit laitier frais qui se place dans la moyenne des produits de sa catégorie en termes de teneurs en protéines. Ainsi, typiquement, un produit initial de la catégorie des yoghourts contiendra en moyenne 4,15% de protéines environ. Un produit initial de la catégorie des yoghourts à boire et de celle des fromages frais contiendra respectivement en moyenne 2,74% et 6,16% de protéines environ.

Par exemple, l'ingestion du produit selon l'invention entraînera chez le consommateur une augmentation de la teneur en protéines de sa prise alimentaire quotidienne d'au moins 17 à 35 g environ, de préférence d'au moins 30 à 35 g environ, dans le cas d'une prise journalière de 2 portions de 125 g chacune (quantité journalière préconisée) d'un produit du type yoghourt ou fromage blanc dont la teneur en protéines totales va de 7 à 13% environ.

La « teneur en protéines totales » ou le « taux protéique » d'un produit correspond à la somme, exprimée en pourcentage, de la concentration du produit en caséines et en protéines sériques. Typiquement, le lait écrémé généralement utilisé par les industriels contient 3,3% de protéines environ.

De plus, on notera que la plupart des ingrédients protéiques laitiers apportent du lactose. Or, pour des raisons de faisabilité technique dans la mesure où le lactose est susceptible d'altérer ou d'inhiber la fermentation, on privilégiera des ingrédients protéiques tels que le produit laitier frais selon l'invention ne contienne pas plus de 11 g environ de lactose pour 100 g. Ainsi, parmi les ingrédients protéiques les plus appropriés dans le cadre de la présente invention,
- l'ingrédient « caséinate de sodium » a l'avantage d'apporter des protéines texturantes sans lactose. Cet ingrédient est constitué en moyenne de 92% de protéines environ ;
- l'ingrédient "poudre de lait écrémé" (ou PLE) est l'ingrédient classique pour apporter des protéines. Cet ingrédient a l'avantage d'être bon marché, mais l'inconvénient d'apporter beaucoup de lactose (composition approximative : 50% de lactose, environ 1/3 de protéines de lait (caséines et protéines sériques) et le reste en sels minéraux et autres) ;
- les protéines sériques particulées permettent d'obtenir une bonne organolepsie. A titre d'exemple, l'ingrédient cité dans les exemples ci-après (Simplesse 100 E) contient environ 50% de protéines.

De manière générale, on notera que la mise en oeuvre de protéines satiétogènes, seuls ou en association, a notamment posé aux Inventeurs des difficultés techniques, difficiles à concilier et à résoudre, aux différents niveaux suivants :
1- la capacité d'écoulement du produit (importante pour le fabricant et pour le consommateur) : il s'agit ici de ce que l'on a décrit plus haut sous le terme de « convivialité » ;
2- l'homogénéité de consistance du produit, reflétant la qualité physique globale du produit fini : absence d'ingrédients coagulés, précipités, agrégés (par exemple en ce qui concerne les protéines qui ont tendance à coaguler en milieu acide et/ou à se dénaturer à chaud, aboutissant à former des agrégats) ; bonne dispersion des différents ingrédients (par exemple lorsque l'on utilise des fibres sous la forme de poudres) ;
3- les propriétés organoleptiques du produit fini, importantes pour le consommateur : texture, appétence et bon goût (absence de goûts « parasites » tels qu'une amertume, une acidité trop importante, une aigreur que l'on peut par exemple observer avec certains ingrédients sous certaines conditions).

Il s'agissait donc pour les Inventeurs de rendre compatibles les objectifs liés aux propriétés organoleptiques et nutritionnelles du produit (pouvoir satiétogène, faible densité énergétique, bon goût, appétence et texture) avec les contraintes de fabrication (faisabilité technologique du produit, aptitude à la fabrication en respect des exigences (i) à (vii) énoncées plus haut). C'est pourquoi, dans le présent contexte, ne sont considérés comme couverts par les termes « produits laitiers frais », utilisés notamment dans les revendications ci-après, que les produits laitiers frais remplissant de manière satisfaisante tous les critères énoncés ci-dessus (de (i) à (vii) et de 1 à 3). Les efforts de recherche des Inventeurs leur ont permis de mettre en évidence que seule la combinaison des caractéristiques de la présente invention permet de remplir l'ensemble de ces critères de manière satisfaisante.

Les protéines satiétogènes utilisées pour enrichir le produit selon la présente invention sont choisies parmi les proteines laitières sériques, le cas échéant les protéines végétales et/ou les protéines laitières choisies parmi la poudre de lait, et les caséines. Les protéines végétales comprennent, par exemple, des protéines de soja. Le produit laitier frais comprend des protéines laitières sériques satiétogènes. Les protéines sériques sont de petites protéines stables en milieu acile mais sensibles au traitement thermique. Dans le cadre de l'invention, on utilise des protéines sériques particulées qui ont pour effet d'améliorer considérablement les propriétés organoleptiques du produit (on utilise, par exemple, les protéines de la marque SIMPLESSE vendue par CP Kelco - Atlanta, GA, Etats-Unis). Ces protéines sériques particulées ont un diamètre d'environ 1µm, ce qui leur permet de mimer les globules gras dans une préparation ou un produit.

Selon un mode de réalisation particulièrement préféré, le produit laitier frais présente, non seulement le facteur d'enrichissement en protéines satiétogènes ci-dessus énoncé, mais aussi un facteur d'enrichissement en protéines sériques satiétogènes allant de 2 à 5 environ, de préférence allant de 3 à 5 environ, avec une valeur encore préférée à 3 environ, par rapport à la teneur en protéines sériques dans le produit initial. Les protéines sériques particulées représentent au moins 16% en poids environ, des protéines satiétogènes que l'on incorpore dans le produit laitier frais selon la présente invention.

Selon un mode de réalisation, le produit laitier frais conforme à la présente invention comprend en outre au moins une protéine satiétogène additionnelle choisie parmi les protéines végétales et de préférence les protéines de soja, la poudre de lait, des caséines, et leurs mélanges.

Selon un mode de réalisation, le produit laitier frais conforme à la présente invention est choisi parmi les yoghourts, les yoghourts à boire, les fromages frais, les laits fermentés.

Avantageusement, la teneur en protéines totales dans ledit produit va :
- de 5 à 20% environ pour un yoghourt ;
- de 3 à 20% environ pour un yoghourt à boire ;
- de 6 à 20% environ pour un fromage frais ;
avec des valeurs plus avantageuses :
- de 6 à 15% environ pour un yoghourt ;
- de 4 à 15% environ pour un yoghourt à boire ;
- de 7 à 15% environ pour un fromage frais ;
et des valeurs préférées :
- de 6 à 7% environ pour un yoghourt ;
- de 4 à 5% environ pour un yoghourt à boire ;
- de 7 à 8% environ pour un fromage frais ;
et des valeurs encore préférées :
- de 6,5% environ pour un yoghourt ;
- de 4,7% environ pour un yoghourt à boire ;
- de 7,5% environ pour un fromage frais.

Selon un mode de réalisation, le produit comprend en outre une ou plusieurs pectines. Plus particulièrement, le produit selon l'invention peut contenir au moins une pectine possédant la propriété d'interagir avec les protéines sériques en milieu acide afin de prévenir ou limiter leur agrégation lors du traitement thermique de la préparation. Une telle pectine est par exemple hautement méthylée. Lorsque le pH de la préparation intermédiaire est inférieur au pHᵢ des protéines, elles sont chargées positivement et peuvent établir des interactions électrostatiques attractives avec les chaînes de pectines chargées négativement.

Selon un autre mode de réalisation, le produit comprend en outre des fibres alimentaires autres que les pectines. Parmi ces fibres, l'on peut citer à titre d'exemples les fibres non-hydrosolubles de fruits et de céréales, les amidons et les maltodextrines résistants, le polydextrose, les fructooligosaccharides (FOS), et leurs mélanges. De plus, l'on peut avantageusement citer les fibres hydrosolubles satiétogènes, qui sont de préférence viscosifiantes. De telles fibres peuvent notamment être choisies parmi les galactomannanes et notamment la gomme de guar, les glucomannanes, les carraghénanes, les alginates, le psyllium, et des combinaisons de ceux-ci.

Par « fibres viscosifiantes », on qualifie usuellement des fibres qui apportent de la viscosité à faible dose. Le terme « fibre » fait référence à des composés non (ou seulement très partiellement) métabolisés, tels que définis plus haut. Parmi ces composés, les polymères de masse molaire élevée sont dits « viscosifiants » dans la mesure où leur incorporation à de faibles doses (typiquement entre 0,05 et 0,5% environ) peut augmenter la viscosité du solvant de plusieurs ordres de grandeur. Cet effet est lié à un gonflement osmotique important de la chaîne de polymère dans le solvant, qui adopte une conformation (plus ou moins) étendue, mobilisant ainsi un nombre important de molécules d'eau. De fait, la solution contenant le polymère viscosifiant (ou épaississant) s'écoule plus lentement, la viscosité étant définie comme le ratio entre la contrainte exercée pour générer l'écoulement et la vitesse caractéristique de cet écoulement. Pour quantifier objectivement le caractère épaississant d'un polymère, il est avantageux de se référer au volume occupé par la chaîne de polymère en solution : la viscosité intrinsèque est par définition le volume dit « hydrodynamique » occupé par gramme de polymère en solution. Typiquement, des guars natifs possèdent une viscosité intrinsèque de l'ordre de 10 dl/g tandis que des guars partiellement hydrolysés mentionnés dans la présente invention ont des viscosités intrinsèques comprises entre 0,3 (Sunfiber^{®}) et 1,0 dl/g (Meyprodor 5) environ. L'apport de viscosité par le polymère peut être caractérisé par le produit de la concentration incorporée (en g/dl) par la viscosité intrinsèque (en dl/g). Ce nombre adimensionnel traduit bien que la viscosité est liée à la fois au volume hydrodynamique du polymère en solution et à la concentration mise en oeuvre. La pertinence de cet invariant pour décrire l'effet épaississant a été montré par de nombreuses équipes travaillant sur la rhéologie des solutions de biopolymères (Robinson et al, 1982, Launay et al., 1986).
Les nombreux essais réalisés par les Inventeurs ont montré que le guar natif, très viscosant, ne peut être incorporé qu'à des doses faibles dans la préparation intermédiaire, c'est-à-dire à des doses ne dépassant pas 1%. En effet, au-delà, la texture de la préparation est filante, viscoélastique, incompatible avec un procédé de fabrication standard (voir définition supra), et ne convient pas au consommateur, qui ne retrouve pas la texture « courte » caractéristique des produits de type yoghourts, fromages ou yoghourts à boire. Inversement, le guar partiellement hydrolysé présente une plus faible viscosité intrinsèque mais peut être incorporé à hauteur de 20% dans une préparation intermédiaire. Au final, les produits [concentration x viscosité intrinsèque] sont comparables, puisque ce produit vaut environ 10 pour le guar natif et est compris entre 6 et 20 pour les guars partiellement hydrolysés mentionnés dans la présente invention. Dès lors, si l'on prend en compte que des quantités incorporées sont élevées, on peut donc bien parler de fibres solubles viscosifiantes, et ce, même avec un polymère partiellement hydrolysé.

De préférence, les fibres alimentaires contenues dans le produit laitier comprennent au moins de la gomme de guar, polysaccharide à masse molaire élevée extrait de l'arbuste Cyamopsis tetragonoloba L. par des procédés de meunerie. Ce galactomannane naturel est composé d'unités mannose (D-mannopyranose) liées en β(1-4) et portant statistiquement en position α(1-6) une unité galactose (D-galactopyranose) pour 2 unités mannose.

Selon un mode de réalisation, ladite gomme de guar est au moins partiellement hydrolysée.

L'on entend ici par « partiellement hydrolysée », que la masse (ou la taille) des chaînes est intermédiaire entre celle du guar natif et celle des résidus sucres qui composent le guar. La masse molaire moyenne du guar natif (Mw) est de l'ordre de 10⁶ g/mol environ, avec une distribution plus ou moins large liée à la variabilité biologique et au procédé d'extraction. La masse moléculaire du monomère de galactose ou de mannose est de 180 g/mol. Les gommes guar partiellement hydrolysées mentionnées dans l'invention ont des masses molaires Mw (mesurées par chromatographie d'exclusion stérique couplée avec réfractométrie et diffusion de la lumière) comprises entre 5000 et 100 000 g/mol environ, de préférence entre 15 000 g/mol et 70 000 g/mol environ : ces valeurs sont intermédiaires entre 180 g/mol et 10⁶ g/mol, d'où l'appellation de guar partiellement hydrolysé (en anglais : PHGG, « partially hydrolysed guar gum »).

Selon un autre mode de réalisation, ladite gomme de guar est neutre en goût.

Dans le cadre de l'invention, une gomme de guar « neutre en goût » n'apporte pas un goût de haricot comme le fait par exemples la gomme de guar native. De plus, elle n'apporte pas un goût acide/aigre tel qu'un goût de vinaigre (ce goût est apporté par le procédé d'hydrolyse chimique de la gomme de guar). En d'autres termes, une gomme de guar "neutre en goût" n'apporte pas d'arrière-goûts indésirables.

Pour obtenir des effets intéressants d'un point de vue nutritionnel, ladite gomme de guar est avantageusement ajoutée à hauteur de 1 à 6 g environ, de préférence à hauteur de 1,5 à 3 g environ, de préférence encore à raison d'au moins 2 grammes environ par portion de produit ingéré. Le terme « portion » désigne ici une unité de conditionnement du produit dans sa forme commercialisée. Par exemple, ce peut être un pot de yoghourt, une bouteille de yoghourt à boire, un pot ou une faisselle de fromage frais...

A des fins de faisabilité technique du produit, on préférera ajouter la gomme de guar, de préférence au moins partiellement hydrolysée, à raison de 0,5 à 8 g environ pour 100 g de produit fini, de préférence à raison de 1 à 3 g environ pour 100 g de produit fini.

Selon un mode de réalisation, le produit laitier comprend en outre d'autres ingrédients choisis parmi des stabilisants, des édulcorants, des arômes, des exhausteurs de goût, des colorants, des agents antimousse, des fruits, etc.

De préférence, les protéines satiétogènes ajoutées à l'étape a) comprennent des protéines sériques de sorte que le facteur d'enrichissement du mélange en protéines sériques satiétogènes va de 2 à 5 environ, de préférence de 3 à 5 environ, avec une valeur toute préférée à 3 environ, par rapport à la teneur en protéines sériques dans le mélange initial.

Dans les procédés objets de la présente invention, on peut mettre en oeuvre de manière équivalente le traitement thermique avant l'homogénéisation ou, inversement, l'homogénéisation avant le traitement thermique. Ces deux ordres d'enchaînement d'étapes sont donc réciproques, alternatifs et équivalents.

De préférence, les procédés selon l'invention comprend en outre au moins une étape de lissage du mélange, réalisée avant et/ou après l'étape d) de refroidissement dudit mélange.

Les procédés selon l'invention peut utiliser, lors de l'étape d'acidification, un ferment comprenant au moins la souche *Streptococcus thermophilus* I-1477, déposée à la CNCM (Institut Pasteur, Paris, France) le 22/09/1994.

De préférence, afin d'obtenir les meilleurs résultats en termes de (α) pouvoir satiétogène, (β) propriétés nutritionnelles et (γ) propriétés de texture et de viscosité du produit, ainsi qu'en terme de (δ) compatibilité du procédé de fabrication du produit avec les contraintes technologiques et industrielles évoquées ci-dessus (cf. les points i) à (vii) et 1 à 3 supra), les protéines satiétogènes que l'on ajoute au mélange laitier comprennent au moins 16% en poids environ, de protéines sériques particulées, le reste (c'est-à-dire au maximum 84% en poids environ) étant essentiellement constitué de poudre de lait et/ou de caséines (par exemple, du caséinate de sodium) et/où de protéines sériquès et/ou de protéines végétales.

Dans les modes de réalisation mettant en oeuvre des fibres alimentaires, notamment de la gomme de guar, on préfère ajouter (soit directement dans le mélange laitier, soit via une préparation intermédiaire) la gomme de guar (en particulier, une gomme de guar au moins partiellement hydrolysée) à raison de 0;5 à 8 g environ pour 100 g de produit fini, de préférence à raison de 1 à 3 g environ pour 100 g de produit fini.

De manière intéressante, les conditions de mise en oeuvre des protéines satiétogènes ont été sélectionnées par les Inventeurs de sorte que ces protéines n'apportent pas une quantité de lactose susceptible d'inhiber la fermentation. Ainsi, les protéines satiétogènes sont avantageusement mis en oeuvre de manière à ne pas dépasser 11 g environ de lactose pour 100 g de produit fini.

Afin d'éviter une détérioration indésirable lors du ou des traitements thermiques, les protéines satiétogènes sont avantageusement utilisées de sorte que le ratio caséines / protéines sériques dans la masse laitière fermentée va de 2,0 à 4,88 environ, de préférence de 2,5 à 3,5 environ et, de manière encore préférée, de 2,8 à 3,3 environ.

Les figures suivantes illustrent des exemples de modes de réalisation de la présente invention :
- Figure 1 : schéma d'un procédé de fabrication d'un produit laitier frais du type yoghourt aux fruits et aux fibres alimentaires, à 0% de sucre ajouté et sans matière grasse ;
- Figure 2 : schéma d'un procédé de fabrication d'un produit laitier frais du type yoghourt aux fruits contenant des fibres alimentaires, à 0% de sucre ajouté et sans matière grasse.

D'autres modes de réalisation et avantages de la présente invention ressortent des exemples suivants, destinés à illustrer, sans pour autant limiter, l'invention.

### EXEMPLES

### 1. Yoghourt aux fruits très enrichi en protéines et contenant des fibres alimentaires

**Tableau 2**

| **Formule à 13% de protéine** | | |
|---|---|---|
| **Composition dans le mélange** | | **Ingrédients** |
| **Masse blanche à 13% de protéines** | **85%** | 13% protéines |
| | | 10% lactose |
| | | 0,3 % lipides |
| | | 76,7% eau |
| **Préparation de fruits à 13% de protéines** | **15%** | 25% purée de pomme (concentration x 1,6) |
| | | 13% NZMP 8899 (12,1% protéine-0,2 matière grasse) |
| | | 11% Sunfiber® |
| | | 0,5% Pectine hautement méthylée |
| | | 0,1% Aspartame |
| | | 0,05% Acesulfame K |
| | | 1,7% d'acide citrique |
| | | 48,65% eau |

avec la composition suivante du mélange laitier (masse blanche) :
- lait écrémé 80,79%
- poudre de lait écrémé (EPI Ingrédients) 9,92%
- Simplesse 100 E (CP Kelco) 3,96%
- Caséinate de sodium (Armor Protéines) 5,32%

### 2. Yoghourt aux fruits et aux fibres alimentaires - 0% sucre ajouté et matière grasses

### 2.1. Exemples de formules finales de yoghourts :

a) Lait écrémé, poudre de lait écrémé, pomme (3,0%), concentrat de protéines de lait, gomme de guar (fibre alimentaire) 1,7 %, céréales (1,5%) (son d'avoine et son de blé), fructose (1,3%), protéines de soja (1,2%), ferments, édulcorants (aspartame & acesulfame K) et arômes.
b) Lait écrémé, poudre de lait écrémé, fruits (1,4% fraise, 0,5% cerise, 0,5% framboise et 0,2% groseille), concentrat de protéines de lait, gomme de guar (fibre alimentaire) 1,7 %, céréales (1,5%)(son d'avoine et son de blé), fructose (1,3%), protéines de soja (1,2%), ferments, édulcorants (aspartame & acesulfame K) et arômes et colorants naturels E-120.

### 2.2. Cas de la formulation a) selon le paragraphe 2.1

### a) Pour 100g de produit fini

**Tableau 3**

| | **% POIDS** |
|---|---|
| Lait écrémé 0.05 % de MG* | 72.414 |
| Poudre de lait écrémé 1 % MG | 5.492 |
| Concentrat de protéines laitières 50 % prot. | 2.025 |
| Crystalline Fructose | 1.053 |
| Acesulfame-k | 0.009 |
| Préparation de Fruits Pomme Céréales | 19.000 |
| Ferments | 0.008 |

| | |
|---|---|
| MG : Matière grasse | |

### b) Composition d'une préparation intermédiaire

**Tableau 4**

| | **Ingrédients** | **Quantité (%)** |
|---|---|---|
| **FRUITS (VEGETAUX/CEREALES)** | Pomme | 16 |
| | - Aseptique / Sous forme congelée | |
| | - Purée | |
| | - Taille < 0.6 mm | |
| | Avoine | 5 |
| | - Déshydraté | |
| | - Son | |
| | - 0.5≤taille≤1.5 mm | |
| | Blé | 3 |
| | - Déshydraté | |
| | - Son | |
| | - 0.5≤Taille≤1.5 mm | |
| **SUCRE et/ou EDULCORANT** | FRUCTOSE | 1.3 |
| | ASPARTAME (E-951) | 0.087 |
| **AUTRES INGREDIENTS** | Eau | |
| | - Dispersion | 53.183 |
| | FIBRE | |
| | - Sunfiber^{®} | 11 |
| | SOJA | |
| | - Protéines de soja | 8 |
| | STABILISANT | |
| | - Pectine | 0.90 |
| | - Gomme guar | 0.30 |
| | REGULATEUR de pH | |
| | - Acide lactique | 0.20 |
| | AROME | |
| | - Pomme | 0.13 |

Cette préparation intermédiaire est formulée de manière à obtenir 2g de gomme guar par pot de 125 g de produit fini.

### c) Caractéristiques cibles d'un produit fini

**Tableau 5**

| | J + 1 |
|---|---|
| PARAMETRES | CIBLE +- TOLERANCE |
| VISCOSITE TA-XT2 | 28.0 + - 5.0 |
| pH | 4.40 +-0.15 |

| | |
|---|---|
| (L) Légal DLC: Date limite de consommation | |

### d) Paramètres d'un produit fini

**Tableau 6**

| ANALYSE | CIBLE | TOLERANCE |
|---|---|---|
| Matière sèche (%) | 18.2 | 17.2 - 19.2 |
| Lipides (%) | 0.19 | 0.05 - 0.25 |
| Protéines (%) | 6.50 | 6.40 - 6.70 |

| | | |
|---|---|---|
| (L) Légal. | | |

Le taux minimum de protéines dans le produit est de préférence de 6 à 7% environ, ce taux étant de manière encore préféré de 6,5% environ.

### e) Exemple d'un procédé de fabrication

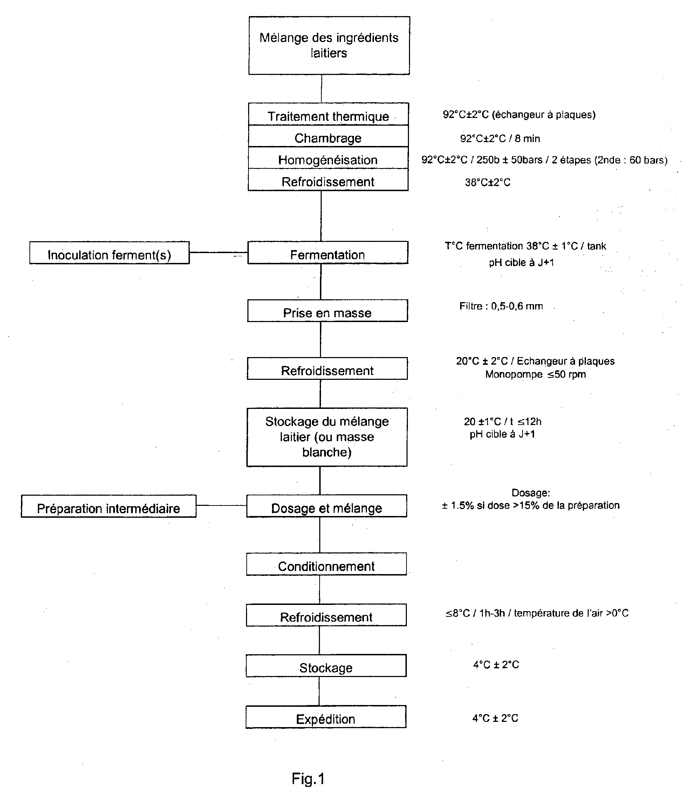

Un exemple de procédé de fabrication est schématisé sur la Figure 1.

### 3. Yoghourt aux fruits contenant des fibres alimentaires - O% sucre ajouté et matière grasse

De la gomme guar est ajoutée directement dans le mélange laitier (ou masse blanche), de manière à obtenir 2g de gomme guar par pot de 170g.

Le produit fini contient de préférence entre 6% et 7% environ de protéines totales.

### 3.1. Composition d'un produit fini

**Tableau 7**

| **Ingrédients** | **%** |
|---|---|
| Lait écrémé standardisé | 82, 547 |
| Alapro 4700 | 1,265 |
| Gélatine, 250 bloom | 0,276 |
| Sunfiber® | 1,500 |
| Fibersol-2 | 1,125 |
| WPC 80 | 1,265 |
| VitA&D | 0,004 |
| Culture | 0,018 |
| Préparation de Fruits & Céréales | 12,000 |
| **Total** | 100 |

### 3.2. Exemple d'un procédé de fabrication

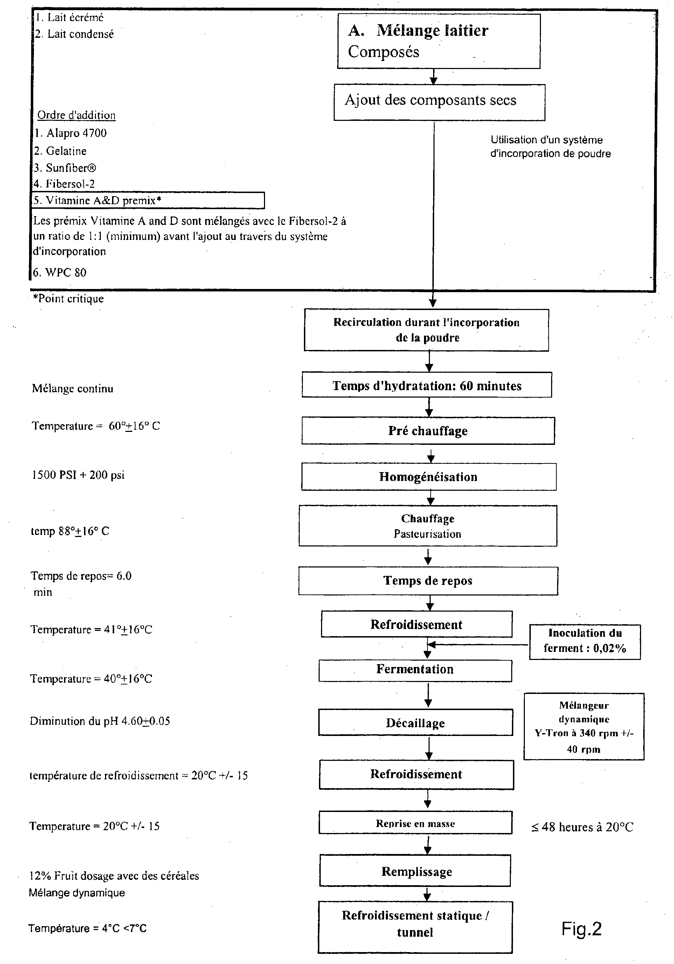

Un exemple de procédé de fabrication est schématisé sur la Figure 2.

### 4. Fromages frais enrichis en protéines et contenant des fibres alimentaires -0% sucre ajouté et matière grasse

Une préparation intermédiaire contenant 5 à 6% environ de protéines laitières sériques déjà acidifiées (Whey Protein Isolate NZMP 8899 - NZMP, Rellingen, Allemagne) est ajoutée dans un mélange laitier du type fromage frais, contenant déjà 8,6% environ de protéines.

Cette préparation intermédiaire est formulée de sorte que 2g de gomme guar sont ajoutés par pot de 150g de produit fini.

Le produit final obtenu contient de préférence 7,5% environ de protéines totales.

### 5. Yoghourt à boire contenant des fibres alimentaires

Le produit final obtenu contient de préférence 4,5% de protéines totales. Le lait est enrichi en protéines en incorporant un mélange de protéines de lait sous forme de poudre (Promilk 602 (INGREDIA))

Une préparation intermédiaire contenant de la gomme guar et, éventuellement des fruits, est ajoutée dans un mélange laitier.

### REFERENCES

Anderson GH, et al. J Nutr. 2004, 134(4):974S-9S.
Araya H, et al. Eur J Clin Nutr 1999, 53(4) : 273-6
Araya H, et al. Int J Food Sci Nutr 2000, 51(2) : 119-24
Bell EA, et al. Am J Clin Nutr 1998, 67 : 412-20
Bensaid A. et al. Physiol Behav 2002, 75: 577-82
Booth DA. Ann NY Acad Sci, 1985; 443: 22-41.
Burton-Freeman B. J Nutr 2000, 130 : 272S-275S
Delargy HJ, et al. Int J Food Sci Nutr 1997, 48(1) : 67-77
De Graaf et al. Am J Clin Nutr 2004, 79:946-61
Green SM, et al. Appetite 1997, 29(3) : 291-304
Green SM, et al. Br J Nutr 2000, 84(4) : 521-30
Holt SH, et al. J Am Diet Assoc 2001, 101(7): 767-73
Howarth NC, et al. Nutr Rev 2001, 59(5) : 129-39
Labouré H, et al. Am J Physiol Regul Integr Comp Physiol 2002, 282(5) : 1501-1511
Louis-Sylvestre J, et al. Neurosci Biobehav Rev, 1980, 47: 608-628.
Mayer J. Glucostatic mechanism of regulation of food intake. N Engl J Med, 1953; 249: 13-16.
Melanson KJ, et al. Am J Physiol, 1999; 277: R337-R345.
Marciani L, et al. Am J Physiol Gastrointest Liver Physiol 2001, 280(6) : G1227-33
Mattes RD & Rothacker D. et al. Physiol Behav 2001, 74(4-5) : 551-7
Poppitt SD et al. Physiol Behav 1998, 64(3) : 279-85
Rolls BJ, et al. Am J Clin Nutr 1999, 70(4) : 448-55
Rolls BJ, et al. Am J Clin Nutr 2000, 72(2) : 361-8
Warwick ZS, et al. Am J Physiol! Regul Integr Comp Physol 2000, 278(1) : R196-200
Westerterp-Plantenga MS, et al. Eur J Clin Nutr 1999, 53(6) : 495-5
G. Robinson, et al. Carbohydrate Research, 107, 17, 1982.
B. Launay, et al. : flow properties of aqueous solutions and dispersions of polysaccharides, Functional Properties of Food Macromolecules, Elsevier Applied Science Pubs, London, 1986.

## Revendications

1. Procédé de fabrication d'un produit aitier frais, comprenant au moins :
a) l'ajout, dans un mélange laitier, d'une ou plusieurs protéines satiétogènes choisies parmi des protéines laitières sériques, et éventuellement des protéines végétales et/ou des protéines laitières choisies parmi la poudre de lait et des caséines, de sorte que
- on ajoute lesdites protéines satiétogènes à hauteur de 2 à 5 fois la quantité de protéines contenues dans le mélange initial ; et
- lesdites protéines satiétogènes contiennent au moins 16% en poids de protéines sériques particulées ayant un diamètre d'environ 1 µm par rapport aux protéines satiétogènes
b) le traitement thermique puis l'homogénéisation du mélange ainsi obtenu ou, réciproquement, l'homogénéisation puis le traitement thermique du mélange ainsi obtenu;
c) la fermentation du mélange issu de l'étape b), ayant pour conséquence son acidification ;
d) le refroidissement du mélange fermenté ; et
e) facultativement, le conditionnement du mélange ainsi obtenu ;
le produit obtenu ne contenant pas plus de
- 0,5 g de sucres pour 100 g de produit s'il est solide ;
- 2,5 g de sucres pour 100 ml s'il est liquide ;
le produit obtenu contenant
- moins de 3 g de matières grasses pour 100 g de produit lorsque le produit est solide ;
- moins de 1 ,5 g de matières grasses pour 100 ml de produit lorsqu'il est liquide ;
le produit obtenu apportant 40 à 120 kcal pour 100g.

2. Procédé selon la revendication 1, **caractérisé en ce que** ledit facteur d'enrichissement en protéines satiétogènes va de 3 à 5 par rapport à la teneur en protéines dans le mélange initial.

3. Procédé selon l'une des revendications précédentes, **caractérisé en ce que** lesdites protéines satiétogènes ajoutées à l'étape a) comprennent des protéines sériques de sorte que le facteur d'enrichissement du mélange en protéines sériques satiétogènes va de 3 à 5, par rapport à la teneur en protéines sériques dans le mélange initial.

4. Procédé selon l'une des revendications précédentes, **caractérisé en ce que** le produit frais ne contienne pas plus de 11 g de lactose pour 100g.

5. Procédé selon l'une des revendications précédentes, **caractérisé en ce que** le produit laitier frais est choisi parmi les yoghourts, les yoghourts à boire, les fromages frais, les laits fermentés.

6. Procédé selon la revendication 5, **caractérisé en ce que** la teneur en protéines totales dans ledit produit va :
- de 5 à 20%, de préférence de 6 à 15%, pour un yoghourt ;
- de 3 à 20% pour un yoghourt à boire ;
- de 6 à 20% pour un fromage frais.

7. Procédé selon l'une quelconque des revendications 1 à 6, **caractérisé en ce que** le produit laitier frais comprend en outre une ou plusieurs pectines.

8. Procédé selon l'une quelconque des revendications 1 à 7, **caractérisé en ce que** le produit laitier frais comprend en outre des fibres alimentaires autres que des pectines.

9. Procédé selon la revendication 8, **caractérisé en ce que** lesdites fibres comprennent des fibres choisies parmi les fibres non-hydrosolubles de fruits et de céréales, les amidons et les maltodextrines résistants, le polydextrose, des fructooligosaccharides, et leurs mélanges.

10. Procédé selon la revendication 8, **caractérisé en ce que** lesdites fibres comprennent des fibres hydrosolubles satiétogènes. de préférence viscosifiantes.

11. Procédé selon la revendication 10, **caractérisé en ce que** lesdites fibres sont choisies parmi des galactomannanes et notamment la gomme de guar, des glucomannanes, les carraghénanes, des alginates, le psyllium, et des combinaisons de ceux-ci.

12. Procédé selon l'une quelconque des revendications 8 à 11, **caractérisé en ce que** lesdites fibres comprennent au moins de la gomme de guar.

13. Procédé selon la revendication 12, **caractérisé en ce que** ladite gomme de guar est au moins partiellement hydrolysée.

14. Procédé selon l'une quelconque des revendications 1 à 13, comprenant en outre au moins une étape de lissage du mélange, réalisée avant et/ou après l'étape d) de refroidissement dudit mélange.

15. Procédé selon l'une quelconque des revendications 1 à 14, dans lequel l'étape d'acidification est réalisée à l'aide d'un ferment comprenant au moins la souche Streptococcus thermophilus I-1477, déposée à la CNCM (Institut Pasteur, Paris, France) le 22/09/1994.

## Patentansprüche

1. Verfahren zur Herstellung eines frischen Milchprodukts, das mindestens umfasst:
a) das Hinzufügen, in ein Milchgemisch, eines oder mehrerer sättigender Proteine, die aus den Milchserumproteinen und eventuell den pflanzlichen Proteinen und/oder den Milchproteinen ausgewählt sind, die aus dem Milchpulver und den Kaseinen ausgewählt sind, so dass
- die sättigenden Proteine in Höhe von 2 bis 5 Mal der Menge der Proteine hinzugefügt werden, die im Ausgangsgemisch enthalten sind, und
- die sättigenden Proteine mindestens 16 Gew.-% partikulierte Serumproteine enthalten, die einen Durchmesser von zirka 1 µm in Bezug auf die sättigenden Proteine haben,
b) das thermische Verarbeiten, gefolgt vom Homogenisieren des derart erhaltenen Gemischs, oder, umgekehrt, das Homogenisieren, gefolgt vom thermischen Verarbeiten des derart erhaltenen Gemischs,
c) das Fermentieren des Gemischs aus Schritt b), was seine Säuerung zur Folge hat,
d) das Abkühlen des fermentierten Gemischs und
e) optional, das Verpacken des derart erhaltenen Gemischs,
wobei das erhaltene Produkt nicht mehr als
- 0,5 g Zucker je 100 g Produkt, wenn es fest ist,
- 2,5 g Zucker je 100 ml, wenn es flüssig ist, enthält,
wobei das erhaltene Produkt enthält
- unter 3 g Fett je 100 g Produkt, wenn es fest ist,
- unter 1,5 g Fett je 100 ml Produkt, wenn es flüssig ist,
wobei das erhaltene Produkt 40 bis 120 kcal je 100 g zuführt.

2. Verfahren nach Anspruch 1, **dadurch gekennzeichnet, dass** der Anreicherungsfaktor mit sättigenden Proteinen von 3 bis 5 in Bezug auf den Gehalt an Proteinen im Ausgangsgemisch reicht.

3. Verfahren nach einem der vorangehenden Ansprüche, **dadurch gekennzeichnet, dass** die in Schritt a) hinzugefügten sättigenden Proteine Serumproteine umfassen, so dass der Anreicherungsfaktor des Gemischs mit sättigenden Serumproteinen von 3 bis 5 in Bezug auf den Gehalt an Serumproteinen im Ausgangsgemisch reicht.

4. Verfahren nach einem der vorangehenden Ansprüche, **dadurch gekennzeichnet, dass** das frische Produkt nicht mehr als 11 g Laktose je 100 g enthält.

5. Verfahren nach einem der vorangehenden Ansprüche, **dadurch gekennzeichnet, dass** das frische Milchprodukt aus den Joghurts, den Trinkjoghurts, den Frischkäsen, der fermentierten Milch ausgewählt ist.

6. Verfahren nach Anspruch 5, **dadurch gekennzeichnet, dass** der Gehalt an Proteinen insgesamt in dem Produkt reicht:
- von 5 bis 20 %, vorzugsweise von 6 bis 15 %, bei einem Joghurt,
- von 3 bis 20 % bei einem Trinkjoghurt,
- von 6 bis 20 % bei einem Frischkäse.

7. Verfahren nach einem der Ansprüche 1 bis 6, **dadurch gekennzeichnet, dass** das frische Milchprodukt ferner ein oder mehrere Pektine umfasst.

8. Verfahren nach einem der Ansprüche 1 bis 7, **dadurch gekennzeichnet, dass** das frische Milchprodukt ferner andere Ballaststoffe als Pektine umfasst.

9. Verfahren nach Anspruch 8, **dadurch gekennzeichnet, dass** die Ballaststoffe Ballaststoffe umfassen, die aus den nicht wasserlöslichen Ballaststoffen von Früchten und von Getreide, den resistenten Stärken und Maltodextrinen, der Polydextrose, den Fructooligosacchariden und ihren Gemischen ausgewählt sind.

10. Verfahren nach Anspruch 8, **dadurch gekennzeichnet, dass** die Ballaststoffe sättigende wasserlösliche, vorzugsweise viskosifizierende, Ballaststoffe umfassen.

11. Verfahren nach Anspruch 10, **dadurch gekennzeichnet, dass** die Ballaststoffe aus den Galactomannanen und insbesondere aus dem Guarkernmehl, den Glucomannanen, den Carrageenen, den Alginaten, dem Psyllium und den Kombinationen derselben ausgewählt sind.

12. Verfahren nach einem der Ansprüche 8 bis 11, **dadurch gekennzeichnet, dass** die Ballaststoffe mindestens Guarkernmehl umfassen.

13. Verfahren nach Anspruch 12, **dadurch gekennzeichnet, dass** das Guarkernmehl mindestens teilweise hydrolysiert ist.

14. Verfahren nach einem der Ansprüche 1 bis 13, umfassend ferner mindestens einen Glättungsschritt des Gemischs, der vor und/oder nach dem Abkühlungsschritt d) des Gemischs durchgeführt wird.

15. Verfahren nach einem der Ansprüche 1 bis 14, wobei der Säuerungsschritt mit Hilfe eines Ferments durchgeführt wird, das mindestens den Stamm Streptococcus thermophilus I-1477, hinterlegt beim CNCM (Institut Pasteur, Paris, Frankreich) am 22.09.1994, enthält.

## Claims

1. A manufacturing process for a fresh dairy product, comprising at least:
a) the addition, to a milk mixture, of one or more satietogenic proteins selected from seric milk proteins, and optionally from vegetable proteins and/or milk proteins selected from milk powder and caseins, such that
- said satietogenic proteins are added at a rate of 2 to 5 times the quantity of proteins contained in the initial mixture; and
- said satietogenic proteins contain at least 16% by weight of particulate seric proteins having a diameter of approximately 1 µm relative to the satietogenic proteins.
b) thermal treatment then homogenisation of the resulting mixture or, reciprocally, homogenisation then thermal treatment of the resulting mixture;
c) fermentation of the mixture coming from step b), resulting in acidification;
d) cooling of the fermented mixture; and
e) optionally, packaging of the resulting mixture;
the product obtained containing no more than
- 0.5 g of sugars per 100 g of product if solid;
- 2.5 g of sugars per 100 ml if liquid;
the product obtained containing
- less than 3 g of fats per 100 g of product when the product is solid;
- less than 1.5 g of fats per 100 ml of product when liquid;
the product obtained contributing from 40 to 120 kcal per 100 g.

2. The process according to claim 1, **characterized in that** said enrichment factor with satietogenic proteins varies from 3 to 5 relative to the content of proteins in the initial mixture.

3. The process according to one of the preceding claims, **characterized in that** said satietogenic proteins added at step a) comprise seric proteins such that the enrichment factor of the mixture with seric satietogenic proteins varies from 3 to 5, relative to the content of seric proteins in the initial mixture.

4. The process according to one of the preceding claims, **characterized in that** the fresh product contains no more than 11 g of lactose per 100 g.

5. The process according to one of the preceding claims, **characterized in that** the fresh dairy product is selected from yoghurts, drinkable yoghurts, fresh cheese, fermented milks.

6. The process according to claim 5, **characterized in that** the content of total proteins in said product varies from:
- 5 to 20%, preferably from 6 to 15%, for yoghurt;
- 3 to 20% for drinkable yoghurt;
- 6 to 20% for fresh cheese.

7. The process according to any one of claims 1 to 6, **characterized in that** the fresh dairy product further comprises one or more pectins.

8. The process according to any one of claims 1 to 7, **characterized in that** the fresh dairy product further comprises dietary fibres other than pectins.

9. The process according to claim 8, **characterized in that** said fibres comprise fibres selected from non-hydrosoluble fibres of fruits and cereals, resistant starches and resistant maltodextrins, polydextrose, fructooligosaccharides, and their mixtures.

10. The process according to claim 8, **characterized in that** said fibres comprise satietogenic hydrosoluble fibres, preferably viscosifying.

11. The process according to claim 10, **characterized in that** said fibres are selected from galactomannanes and especially guar gum, glucomannanes, carrageenans, alginates, psyllium, and combinations thereof.

12. The process according to any one of claims 8 to 11, **characterized in that** said fibres comprise at least guar gum.

13. The process according to claim 12, **characterized in that** said guar gum is at least partially hydrolysed.

14. The process according to any one of claims 1 to 13, further comprising at least one smoothing step of the mixture, conducted prior to and/or after the cooling step d) of said mixture.

15. The process according to any one of claims 1 to 14, wherein the acidification step is conducted by means of a ferment comprising at least the Streptococcus thermophilus I-1477 strain, filed with the CNCM (Institut Pasteur, Paris, France) on 22/09/1994.
